# EUROPÄISCHE PATENTSCHRIFT

(11) **EP 2 968 796 B1**
(45) Veröffentlichungstag und Bekanntmachung des Hinweises auf die Patenterteilung: **09.05.2018**
(21) Anmeldenummer: 14707783.8
(22) Anmeldetag: 04.03.2014
(51) Int. Cl.: A61M 5/34

(54) **AUFSATZ FÜR EINE SPRITZE ODER KARPULE**
ATTACHMENT FOR A SYRINGE OR CARPULE
EMBOUT POUR UNE SERINGUE OU CARPULE

(30) Priorität: 11.03.2013 DE 102013204134
(43) Veröffentlichungstag der Anmeldung: 20.01.2016
(73) Patentinhaber: Vetter Pharma-Fertigung GmbH & Co. KG, 88212 Ravensburg (DE)
(72) Erfinder: ZENKER, Jochen, 88212 Ravensburg (DE)
(74) Vertreter: Gleiss, Alf-Olav
(86) Internationale Anmeldenummer: PCT/EP2014/054142
(87) Internationale Veröffentlichungsnummer: WO 2014/139832

(56) Entgegenhaltungen:
- DE-A1-102009 007 250

## Beschreibung

Die Erfindung betrifft einen Aufsatz für eine Spritze oder eine Karpule gemäß Oberbegriff des Anspruchs 1.

Aufsätze für Spritzen, Karpulen oder dergleichen sind bekannt (DE 10 2009 007 250 A1). Sie werden auch als Adapter verwendet und dienen dazu, ein Befestigungsmittel für eine Kanüle oder eine sonstige Vorrichtung, beispielsweise eine Injektionseinrichtung oder dergleichen bereitzustellen. Sie werden auf einen Fortsatz der Spritze, Karpule oder dergleichen aufgesetzt und befestigt. Sie weisen in der Regel ein Innengewinde auf, in welches ein Ansatz einer Kanüle oder dergleichen eingeschraubt werden kann. Häufig sind Aufsätze der hier angesprochenen Art auch als Sicherheits- oder Garantieverschluss ausgebildet und weisen ein Befestigungsteil auf, welches fest auf einem endständigen Fortsatz einer Spritze, einer Karpule oder dergleichen aufgesetzt und dort verriegelnd gehalten wird. Über eine Sollbruchlinie ist mit dem Befestigungsteil bei derartigen Verschlüssen eine Kappe verbunden, welche das freie Ende des Fortsatzes, der von der Spritze, Karpule oder dergleichen weggerichtet ist, sicher abdeckt, wodurch auch der Innenraum der Spritze, Karpule oder dergleichen geschützt ist. Wird die Kappe abgenommen, reißt die Sollbruchlinie auf, sodass irreversibel erkennbar ist, dass die Kappe entfernt wurde. Damit wird auch ein Schutz vor Manipulationen an dem Aufsatz gewährleistet. Der Fortsatz weist ein freies Ende auf, außerdem vorzugsweise in einem Abstand zu diesem mindestens eine in die Außenfläche des Fortsatzes eingebrachte Vertiefung, die vorzugsweise als Ringnut ausgebildet ist. In diese greift der an der Spritze, Karpule oder dergleichen befestigte Aufsatz verriegelnd ein. Die hier angesprochene Erfindung betrifft sowohl Aufsätze mit einer Sicherheitskappe als auch solche ohne eine derartige Originalitätssicherung. Es hat sich herausgestellt, dass Aufsätze der hier angesprochenen Art häufig bei Einwirkung eines Drehmoments eine Relativdrehung gegenüber dem Fortsatz der Spritze, Karpule oder dergleichen durchführen und sich dabei auch ablösen können, sodass das im Inneren der Spritze, Karpule oder dergleichen vorhandene Medium verunreinigt werden kann und unbrauchbar ist. Dies kann zu hohen finanziellen Verlusten oder aber zu einer Gefährdung von Patienten führen.

Aufgabe der Erfindung ist es daher, einen Aufsatz der hier angesprochenen Art so auszubilden, dass er sicher an einer Spritze oder Karpule angebracht werden kann, wobei mit hoher Sicherheit eine Relativdrehung zwischen Aufsatz und Spritze oder Karpule vermieden wird.

Zur Lösung dieser Aufgabe wird ein Aufsatz der hier angesprochenen Art vorgeschlagen, welcher die in Anspruch 1 aufgeführten Merkmale umfasst. Der so realisierte Aufsatz weist einen einen Innraum umschließenden Grundkörper mit einem Mantel auf sowie einen mit diesem verbundenen, koaxial angeordneten Innenkörper. Bei dem Aufsatz ist vorgesehen, dass eine Innenwand des Innenkörpers, die in aufgesetztem Zustand des Aufsatzes an dem Fortsatz einer Spritze oder Karpule anliegt, über eine Anzahl von Stegen mit dem Mantel des Aufsatzes verbunden ist. Der Aufsatz zeichnet sich dadurch aus, dass zumindest einer der Stege mit einer gedachten, radial verlaufenden Linie, die ein Ende des Steges schneidet oder berührt, einen Winkel einschließen. Dieser ist größer als 0° und spitz. Wird in einen derartigen Aufsatz ein Drehmoment eingeleitet, so verschwenkt ein unter einem Winkel zu einer gedachten radialen Linie verlaufender Steg derart, dass der Steg radial verläuft und damit zwischen dem Mantel und der Innenwand des Innenkörpers gestaucht wird. Dies führt dazu, dass die Innenwand mit einer erhöhten Kraft gegen den Fortsatz der Spritze, Karpule oder dergleichen angepresst und sicher gehalten wird, sodass eine Verdrehung des Aufsatzes gegenüber dem Fortsatz mit hoher Sicherheit vermieden wird.

Ein bevorzugtes Ausführungsbeispiel des Aufsatzes zeichnet sich durch mindestens ein Widerlager auf, welches von der Innenseite des Mantels entspringt und in einem Abstand zu einem Steg angeordnet ist. Dabei ist das Widerlager so angeordnet, dass ein Steg aus seiner Lage, in welche er unter einem spitzen Winkel zu einer gedachten radialen Linie angeordnet ist, maximal bis in eine Position verschwenkt werden kann, wenn ein Drehmoment auf den Aufsatz wirkt, in welcher er radial verläuft und damit eine maximale Anpresskraft auf den Fortsatz ausübt. Würde er über die radiale Linie hinweg verschwenken, so führte dies zu einer Reduktion der Kräfte, welche die Innenwand gegen den Fortsatz anpressen. Durch das Widerlager wird dies verhindert, sodass bei einem auf dem Aufsatz einwirkenden Drehmoment Stege bis zu ihrer eine maximale Anpresskraft wirkenden Position verschwenkt werden können und dann durch das Widerlager in dieser Position gehalten werden.

Ein weiteres bevorzugtes Ausführungsbeispiel zeichnet sich dadurch aus, dass das Widerlager einen seitlichen Vorsprung aufweist, der sich in Umfangsrichtung in Richtung auf den zugehörigen Steg erstreckt und damit eine definierte Anlageposition des Stegs am Widerlager bewirkt. Damit lassen sich die auf einen Steg wirkenden Kräfte vorherbestimmen, sodass das Verhalten eines Aufsatzes bei Einleitung eines Drehmoments genau vorherbestimmt werden kann.

Bei einem weiteren bevorzugten Ausführungsbeispiel sind zwei Widerlager vorgesehen, sodass auch mehr als ein Steg daran gehindert werden kann, über die radiale Position verschwenkt zu werden. Denkbar ist es aber auch, Widerlager so anzuordnen, dass sie Stege eines Aufsatzes gegen ein ungewolltes Verdrehen in beiden Richtungen abstützen.

Weitere Ausgestaltungen des Aufsatzes ergeben sich aus den Unteransprüchen.

Die Erfindung wird im Folgenden anhand der Zeichnung näher erläutert. Es zeigen:
- Figur 1: eine Prinzipskizze eines ersten Ausführungsbeispiels eines Aufsatzes in perspektivischer Unteransicht;
- Figur 2: eine Prinzipskizze eines zweiten Ausführungsbeispiels des Aufsatzes in Unteransicht;
- Figur 3: eine perspektivische Innenansicht des in Figur 2 wiedergegebenen Aufsatzes; und
- Figur 4: eine Innenansicht eines dritten Ausführungsbeispiels eines Aufsatzes.

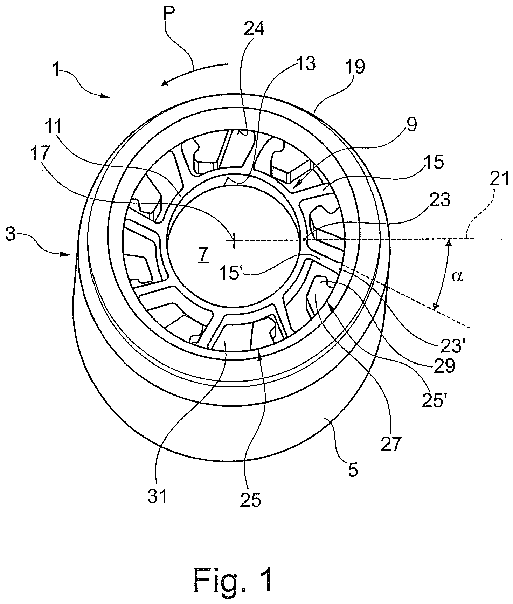

Das in Figur 1 dargestellte erste Ausführungsbeispiel eines Aufsatzes 1 ist für Spritzen oder Karpulen geeignet und weist einen Grundkörper 3 auf, dessen Mantel 5 einen Innenraum 7 umgibt. Mit dem Mantel 5 ist ein Innenkörper 9 verbunden, welcher eine Innenwand 11 aufweist, die mit ihrer Innenfläche 13 in aufgesetztem Zustand des Aufsatzes 1 auf einen Fortsatz einer Spritze, Karpule oder dergleichen mit dessen Außenfläche in Berührung tritt. Die Innenwand 11 des Innenkörpers 9 ist über eine Anzahl von Stegen 15 mit dem Mantel 5 verbunden, wobei die Stege von der Innenseite des Mantels 5 entspringen. Ihr Innenkörper 9 ist also koaxial zum Mantel 5 angeordnet.

Der Grundkörper 3 des Aufsatzes 1 weist eine Mittelachse 17 auf, die senkrecht auf einer gedachten Ebene steht, in welcher die in Figur 1 dem Betrachter zugewandte Unterseite 19 des Aufsatzes 1 liegt. Durch die Mittelachse 17 verläuft eine gedachte radial verlaufende Linie 21, die auch durch ein Ende eines Steges 15 oder durch einen Übergangspunkt 23 zwischen einem Ende eines Steges 15 und der Innenwand 11 verläuft. Mit dieser Linie 21 schließt mindestens ein zwischen Mantel 5 und Innenkörper 9 verlaufender Steg 15 einen Winkel α ein, der größer als 0° und spitz ist. Dieser Winkel öffnet sich von dem Übergangspunkt 23 nach außen, derart, dass eine konkreter Steg 15' in Figur 1 unterhalb der radial verlaufenden Linie 21 angeordnet ist. Bei dem in Figur 1 dargestellten Ausführungsbeispiel sind alle Stege des Aufsatzes 1 so gegenüber einer gedachten radial verlaufenden Linie 21 angeordnet, dass sie die entsprechende Orientierung wie der Steg 15' aufweisen.

Das in Figur 1 dargestellte Ausführungsbeispiel zeichnet sich dadurch aus, dass die Innenwand 11 durchgehend ausgebildet ist, also einen Ring darstellt, der von den Stegen 15 in einem Abstand gegenüber dem Mantel 5 des Grundkörpers 3 des Aufsatzes 1 gehalten wird.

Der durch die Innenfläche 13 der Innenwand 11 definierte Innendurchmesser des Innenkörpers 9 ist etwas kleiner als der Außendurchmesser des Fortsatzes einer Spritze, Karpule oder dergleichen, auf den der Aufsatz 1 aufgesetzt werden soll. Beim Aufsetzen des Aufsatzes 1 auf den hier nicht dargestellten Fortsatz wird also der Innenkörper 9 aufgeweitet. Sollte, wie es vorzugsweise vorgesehen ist, der Fortsatz im Bereich seiner Außenfläche eine vorzugsweise ringförmige Vertiefung aufweisen, so schnappt die Innenfläche 13 der Innenwand 11 beim Aufsetzen des Aufsatzes 1 auf einen Fortsatz in diese Ringnut ein und hält den Aufsatz 1 sicher an dem Fortsatz der Spritze, Karpule oder dergleichen.

Mindestens einem der Stege 15 ist ein Widerlager 25 zugeordnet, welches von der Innenseite 24 des Mantels 5 entspringt und sich in Richtung des Innenraums 7 erstreckt. Das Widerlager 25 weist einen Basiskörper 27 auf, der sich ausgehend von der Innenseite 24 in Richtung auf sein gegenüberliegendes freies Ende vorzugsweise verjüngt und weist auf einer Seite einen vorzugsweise am freien Ende entspringenden Vorsprung 29 auf, der sich in Richtung auf den benachbarten Steg, hier in Richtung auf den Steg 15' erstreckt.

Bei dem hier dargestellten Ausführungsbeispiel des Aufsatzes 1 ist jedem Steg 15 ein derartiges Widerlager 25 zugeordnet.

Durch zwei benachbarte Stege 15 und einen zugehörigen Bereich der Innenwand 11 werden Freiräume 31 geschaffen, die von der Innenseite 24 des Mantels 5 und den dem Freiraum 31 zugewandten Innenflächen der Stege sowie der Innenwand 11 umschlossen werden.

Bei dem in Figur 1 dargestellten Ausführungsbeispiel sind acht in gleichem Umfangsabstand zueinander angeordnete, in gleicher Winkellage zu einer gedachten radial verlaufenden Linie 21 angeordnete Stege 15 vorgesehen, zwischen denen - in Umfangsrichtung gesehen - jeweils ein Freiraum 31 gebildet wird, der radial außen von der Innenseite 24 des Mantels 5 und radial innen von der Innenseite der Innenwand 11 und schließlich von den Innenflächen der benachbarten Stege 15 begrenzt wird. Hier ist vorzugsweise in jedem Freiraum 31 ein Widerlager 25 vorgesehen, welches einen Basiskörper 27 und einen Vorsprung 29 aufweist, wie dies oben beschrieben wurde.

Das hier beschriebene Ausführungsbeispiel kann dadurch abgewandelt werden, dass nur zwei Widerlager 25 vorgesehen werden, oder dass nur jedem zweiten Freiraum 31 ein Widerlager zugeordnet ist oder dergleichen.

Darüber hinaus ist es möglich, die Innenwand 11 nicht als geschlossenen Ring auszubilden, sondern Ringsegmente vorzusehen, die von zwei unmittelbar benachbarten Stegen 15 und einem Abschnitt der Innenwand 11 gebildet werden, der die beiden Stege verbindet. Rechts und links von den durch einen Abschnitt der Innenwand 11 verbundenen Stegen ist dann keine Innenwand vorgesehen. Die rechts und links nachfolgenden Stege bilden dann mit den jeweils angrenzenden Stegen und einem diesen zugeordneten Abschnitt der Innenwand weitere Ringsegmente. In anderen Worten: Die in Figur 1 durchgehende Innenwand 11, die alle Freiräume 31 überspannt, entfällt bei einem abgewandelten Ausführungsbeispiel bei jedem zweiten Freiraum, sodass dann bei einem Aufsatz 1 mit acht Stegen 15 vier Ringsegmente gebildet werden. Ein Ringsegment kann auch zwei benachbarte Freiräume 31 einschließen, wobei jeder der Freiräume von zwei benachbarten Stegen seitlich begrenzt ist, und wobei der mittlere Steg beiden Freiräumen zugewandt ist.

Bei einer derartigen Ausgestaltung ist es möglich, Widerlager 25 zwischen zwei benachbarten Ringsegmenten vorzusehen und die Freiräume 31 ohne derartige Widerlager 25 auszubilden. Weiterhin ist es möglich, das in Figur 1 dargestellte Ausführungsbeispiel dadurch abzuwandeln, dass vier Ringsegmente gebildet werden, die Freiräume 31 umschließen, und dass nur in diesen umschlossenen Freiräumen 31 Widerlager vorgesehen sind.

Figur 1 lässt erkennen, dass die Widerlager 25 in den Freiräumen 31 nicht mittig angeordnet sind. Vielmehr ist vorgesehen, dass die Widerlager 25 gegenüber einer gedachten Mittellinie eines Freiraums 31 - in Figur 1 nach rechts - versetzt angeordnet sind. Das spezielle Widerlager 25' liegt also näher an dem Steg 15'.

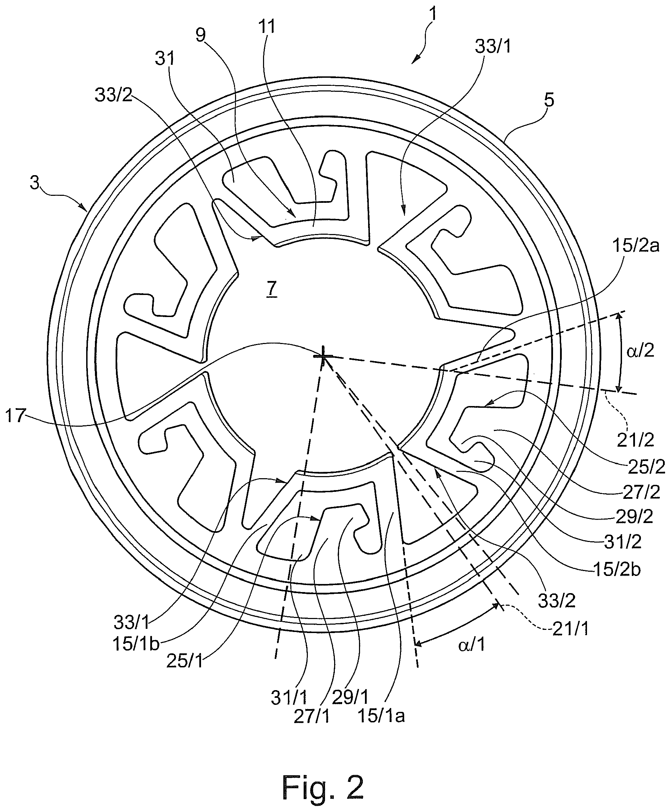

Figur 2 zeigt in Unteransicht ein zweites Ausführungsbeispiel eines Aufsatzes 1. Gleiche Teile sind mit gleichen Bezugsziffern versehen, sodass insofern auf die vorangegangene Beschreibung verwiesen wird.

Es wird deutlich, dass bei dem Ausführungsbeispiel gemäß Figur 2 der Innenkörper 9 keine durchgehende, also ringförmige Innenwand 11 aufweist, sondern dass einzelne Ringsegmente 33 gebildet werden, die jeweils einen Freiraum 31 einschließen. Das Ausführungsbeispiel des in Figur 2 dargestellten Aufsatzes 1 umfasst zwei Arten von Ringsegmenten. Ein erster Typ Ringsegment 33/1 weist einen Steg 15/1a auf, der mit einer gedachten radial verlaufenden Linie 21/1 einen Winkel α/1 einschließt, der sich, von der Mittelachse 17 aus gesehen, nach außen öffnet. Dabei liegt der Steg 15/1 quasi links von der Linie 21/1.

Bei einem anderen Typ Ringsegment 33/2 weist der zugehörige Steg 15/2 gegenüber einer gedachten radial verlaufenden Linie 21/2 in eine Richtung und schließt mit dieser Linie 21/2 einen Winkel α/2 ein, der sich, von der Mittelachse 17 aus gesehen, nach außen öffnet. Hier liegt aber der Steg 15/2 quasi rechts von der Linie 21/2.

Der Freiraum 31/1 des ersten Ringsegments 31/1 wird auf der dem Steg 15/1a gegenüberliegenden Seite von einem Steg 15/1b begrenzt, der mit dem Steg 15/2b, der dem Steg 15' in Figur 1 entspricht, einen Winkel einschließt, sodass er wiederum quasi links von einer gedachten radial verlaufenden Linie verläuft. Entsprechend wird ein Freiraum 31/2 des Ringsegments 33/2 von einem dem Steg 15/2a gegenüberliegenden Steg 15/2b begrenzt, welcher gegenüber einer gedachten radial verlaufenden Linie einen Winkel einschließt, sodass dieser Steg 15/2b quasi rechts von der gedachten Linie angeordnet ist.

Bei dem in Figur 2 dargestellten Ausführungsbeispiel ist in jedem der Ringsegmente 33/1, 33/2 umschlossenen Freiräume 31/1 und 31/2 ein Widerlager vorgesehen, wobei ein Widerlager 25/1 dem Steg 15/1a und ein Widerlager 25/2 dem dem Steg 15/2a gegenüberliegenden Steg 15/2b zugeordnet ist. Die beiden Widerlager 25/1 und 25/2 sind also wieder gegenüber einer gedachten Mittellinie der Freiräume versetzt angeordnet.

Beide Widerlager 25/1 und 25/2 sind identisch aufgebaut, wie dies anhand von Figur 1 erläutert wurde. Sie weisen also einen Basiskörper und einen seitlich davon ausgehenden Vorsprung auf, wobei das Widerlager 25/1 einen Basiskörper 27/1 aufweist, von dem ein Vorsprung 29/1 ausgeht, der gegen den Uhrzeigersinn vorspringt. Das Widerlager 25/2 ist spiegelbildlich angeordnet: Es weist einen Basiskörper 27/2 auf, von dem ein Vorsprung 29/2 nach links vorspringt, also im Uhrzeigersinn.

Zwischen den Ringsegmenten 31/1 und 31/2 sind jeweils Lücken vorgesehen, wobei jeweils eine der Lücken sich V-förmig in den Innenraum 7 öffnet, während sich die andere ausgehend von der Innenwand der Ringsegmente V-förmig sich in Richtung auf den Mantel 5 öffnet. In die letztere der beiden Lücken, also in jene, die sich V-förmig in Richtung auf den Mantel 5 des Grundkörpers 3 öffnen, kann ebenfalls ein Widerlager eingebracht werden, welches einen Vorsprung aufweist, der entweder einem Steg 15/1a oder 15/2b zugeordnet ist. Bei entsprechend groß ausgebildeter Lücke können auch zwei spiegelbildlich zueinander angeordnete Widerlager vorgesehen werden. Derartige Widerlager würden also die zugehörigen Stege, nicht wie in Figur 2 dargestellt, aus dem Freiraum 31 heraus stützen, sondern von außen aus dem Zwischenraum beziehungsweise den Lücken zwischen zwei benachbarten Ringsegmenten.

Bei dem in Figur 2 dargestellten Ausführungsbeispiel weist der Aufsatz 1 zwölf Stege auf, die insgesamt sechs Ringsegmenten zugeordnet sind.

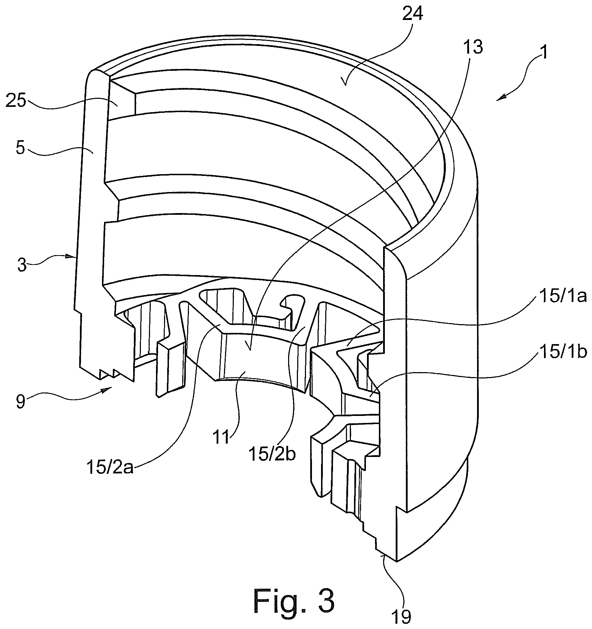

Figur 3 zeigt in perspektivischer Darstellung einen Längsschnitt durch den in Figur 2 dargestellten Aufsatz 1. Gleiche Teile sind mit gleichen Bezugsziffern versehen, sodass insofern auf die vorangegangene Beschreibung verwiesen wird, um Wiederholungen zu vermeiden.

Figur 3 zeigt den Grundkörper 3 des Aufsatzes mit dem Mantel 5 und mit dem davon entspringenden Innenkörper 9, der von der Innenseite des Mantels 5 entspringende Stege 15/1a, 15/1b, 15/2a und 15/2b aufweist, zwischen deren Enden Segmente der Innenwand 11 liegen, die mit ihrer Innenfläche 13 im aufgesetzten Zustand des Aufsatzes 1 an der Außenfläche eines Fortsatzes einer Spritze, Karpule oder dergleichen anliegen.

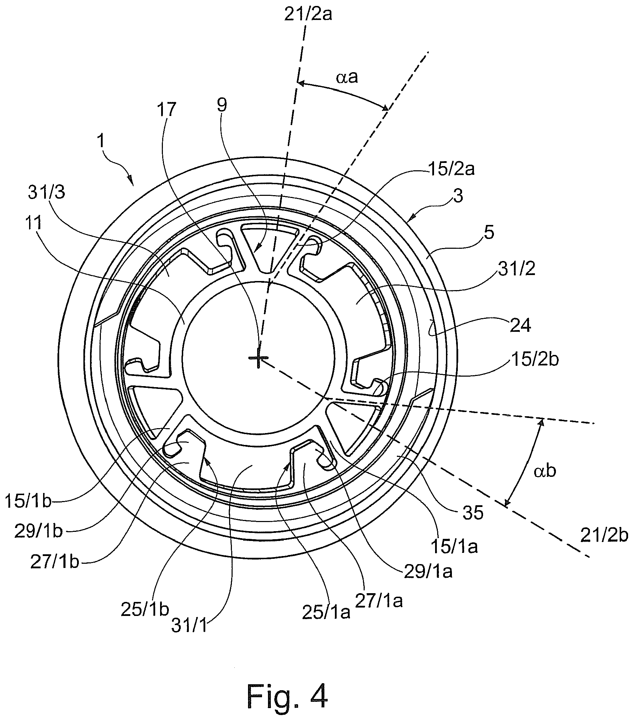

Die Schnittdarstellung gemäß Figur 3 lässt erkennen, dass die in senkrechter Richtung, also in Richtung der hier nicht wiedergegebenen Mittelachse 17 des Aufsatzes 1 gemessene Höhe des Innenkörpers 9 wesentlich geringer ist als die des Mantels 5, der auf seiner Innenfläche 24 mit einem Gewinde 35 versehen ist, das oben in der Beschreibungseinleitung bereits erwähnt wurde und auf das anhand von Figur 4 noch einmal näher eingegangen wird.

Der Innenkörper 9 ist am unteren Ende des Aufsatzes 1, also nahe der Unterseite 19 angeordnet, weil dies für die Befestigung des Aufsatzes 1 an Fortsätzen von herkömmlichen Spritzen, Karpulen oder dergleichen sich als vorteilhaft erwiesen hat. Bei Bedarf kann der Innenkörper 9 auch an einem Abstand zur Unterseite 19 nach oben versetzt am Mantel 5 vorgesehen werden.

Die Höhe des Innenkörpers 9 wird so gewählt, dass beim Aufsetzen des Aufsatzes auf einen Fortsatz und einem Versuch, diesen gegenüber dem Fortsatz zu verdrehen, ausreichend hohe Kräfte aufgebaut werden können.

Figur 3 zeigt schließlich, dass der Innenkörper 9 und der Mantel 5 koaxial in einer Ebene liegen, die parallel zur Unterseite 19 verläuft.

Figur 4 zeigt ein drittes Ausführungsbeispiel eines Aufsatzes 1 in Draufsicht von oben. Wiederum sind gleiche Teile mit gleichen Bezugsziffern versehen, sodass insofern auf die Beschreibung der vorangegangenen Figuren verwiesen wird.

Der Aufsatz 1 weist einen Grundkörper 3 mit einem Mantel 5 auf, an dessen Innenseite 24 ein Gewinde 35 vorgesehen ist, welches mit einem Außengewinde an dem Ansatz einer Kanüle oder eines anderen Elements zusammenwirken kann. Vorzugsweise sind alle Ausführungsbeispiele des Aufsatzes, die hier erläutert wurden, mit einem derartigen Innengewinde versehen. Denkbar ist es aber auch, eine Bajonett-Verriegelung oder ähnliches für Kanülen, Ansätze von Infusionseinrichtungen oder dergleichen vorzusehen.

Der Aufsatz 1 weist einen Innenkörper 9 auf, der mit einer Innenwand 11 versehen ist, die hier durchgehend ausgebildet, also als Ring realisiert ist. Die Innenwand 11 ist über Stege 15 mit dem koaxial zum Innenkörper 9 angeordneten Mantel 5 verbunden, wie dies auch bei den vorangegangenen Ausführungsbeispielen der Fall war.

Bei dem in dieser Figur dargestellten Ausführungsbeispiel sind sechs Stege vorgesehen, die einander paarweise zugeordnet, wobei die Paare in gleichem Umfangsabstand zueinander angeordnet sind. Ein erster Freiraum 31/1 wird von zwei Stegen 15/1a und 15/1b begrenzt, sodass zwischen diesen, der Innenseite 24 des Mantels 5 und der Innenwand 11 der Freiraum 31/1 eingeschlossen ist.

Der Aufsatz 1, wie er in Figur 4 dargestellt ist, weist drei Freiräume 31/1, 31/2 und 31/3 auf, die alle identisch ausgebildet sind, wie der eben erwähnte Freiraum 31/1. Versetzt zu einer gedachten Mittelebene der Freiräume, die hier kurz nur mit 31 bezeichnet werden, sind jeweils den begrenzenden Stegen 15/1a und 15/1b zugeordnete Widerlager 25/1a und 25/1b zugeordnet, die zu der gedachten Mittelebene spiegelbildlich ausgerichtet sind: das Widerlager 25/1a weist einen vom Basiskörper 27/1a nach rechts vorspringenden Vorsprung 29/1a auf, während das Widerlager 25/1b einen vom Basiskörper 27/1b nach links vorspringenden Vorsprung 29/1b aufweist. Die beiden Vorsprünge weisen in Richtung der zugeordneten Stege, das heißt, der Vorsprung 29/1a weist zum Steg 15/1a und der Vorsprung 29/1b zum Steg 15/1b.

Aus der Darstellung des Freiraums 31/2 ist ersichtlich, dass der eine den Freiraum 31/2 begrenzende Steg 15/2a mit einer gedachten radial verlaufenden Linie 21/2a einen Winkel α/a einschließt, der sich ausgehend von der Mittelachse 17 aus gesehen nach außen öffnet, wobei der Steg 15/2a der gedachten Linie 21/2a - im Uhrzeigersinn gesehen - quasi voreilt.

Entsprechend schließt der zweite den Freiraum 31/2 einschließende Steg 15/2b mit einer gedachten radial verlaufenden Linie 21/2b einen Winkel α/b ein, der sich von der Mittellinie 17 aus gesehen nach außen öffnet, wobei der Steg 15/2b - im Uhrzeigersinn gesehen - der Linie 21/2b quasi nacheilt.

Zwischen den Freiräumen sind Lücken, die kreissegmentartig, das heißt im Wesentlichen dreieckförmig, ausgebildet sind, wobei die Basis dieses Dreiecks mit der Innenseite 24 des Mantels 5 zusammenfällt. Die dem Mantel 5 abgewandte Spitze des Dreiecks wird durch die Innenwand 11 geschlossen. Bei einem abgewandelten Ausführungsbeispiel des in Figur 4 dargestellten Aufsatzes 1 könnte die Innenwand 11 hier auch unterbrochen sein, sodass drei identische, separate Ringsegmente gebildet werden.

Im Folgenden wird auf die Funktion eines Aufsatzes 1 näher eingegangen:
Zunächst wird auf das Ausführungsbeispiel gemäß Figur 1 Bezug genommen:
Ein Aufsatz 1 wird auf einen Fortsatz einer Spritze, Karpule oder dergleichen aufgesetzt, sodass er über den Innenkörper 9 sicheren Halt an diesem findet. Die Innenfläche 13 der Innenwand 11 des Innenkörpers 9 liegt fest auf der Außenfläche des Fortsatzes an, sie schnappt vorzugsweise in eine dort vorgesehene Vertiefung, die insbesondere als umlaufende Rille ausgebildet ist. Die Innenwand 11 wird beim Aufsetzen auf den Fortsatz gedehnt, sodass Reibungskräfte zwischen der Innenfläche 13 und der Außenfläche des Fortsatzes wirken.

Wird nun der Aufsatz 1 gemäß der Darstellung in Figur 1, wie durch einen Pfeil P angedeutet, gegen den Uhrzeigersinn gegenüber einem hier nicht dargestellten, die Innenwand 11 durchdringenden Fortsatz gedreht, so wird die Innenwand 11 aufgrund der oben beschriebenen Reibungskräfte festgehalten, während der Mantel 9 sich in Richtung des Pfeils gegen den Uhrzeigersinn bewegt. Der Steg 15' schwenkt dadurch, wie alle anderen Stege auch, um den Übergangspunkt 23 gegen den Uhrzeigersinn. Gleichzeitig schwenkt dieser Steg 15' im Bereich seines Ursprungs 23', also in dem Bereich, in dem der Steg 15' auf der Innenseite 24 des Mantels 9 entspringt. Durch die Schwenkbewegung des Stegs 15' wird dieser so verlagert, dass er gegenüber der Mittelachse 17 in radialer Richtung verläuft. Der Übergangspunkt 23 und der Ursprung 23' bilden also quasi Lagerpunkte des Stegs 15', um dieser bei einer Relativdrehung des Mantels 5 gegenüber der Innenwand 11 des Innenkörpers verschwenkt wird. Dabei wird der Steg 15', der aus einem längsstabilen Material besteht, gestaucht, sodass die Innenwand 11 mit hoher Kraft gegen die Außenfläche eines Fortsatzes angedrückt und damit an diesem drehfest gehalten wird.

Dasselbe gilt für alle Stege 15 des Aufsatzes 1, die sich wie ein Gelenkstab verhalten und dazu dienen, die Innenwand 11 des Innenkörpers 9 durch hohe Reibungskräfte an einem Vorsprung drehsicher zu halten. Um die für die Stauchung der Stege erforderlichen Kräfte aufbauen zu können, ist der Mantel 5 des Aufsatzes 1 stabil ausgebildet.

Wenn die Stege gegenüber der Mittelachse 17 in eine radiale Position verschwenkt sind, legen sich die Widerlager 25 an die Stege 15 an, sodass eine weitere Drehbewegung des Mantels 5 gegenüber der Innenwand 11 verhindert wird.

Um diese Funktionsweise zu realisieren, sind die Stege 15 aus einem festen Material hergestellt, sodass diese möglichst gestaucht werden können, ohne dass ihre Länge wesentlich reduziert wurde. Dabei bauen sie maximale Anpresskräfte auf, während sie aus der in Figur 1 dargestellten Position in ihre radiale Lage verschwenkt werden.

Um hohe Reibungskräfte zwischen der Innenfläche 13 der Innenwand 11 und einem Fortsatz aufzubauen, auf den der Aufsatz 1 aufgesteckt wird, weist die Innenfläche 13 zumindest bereichsweise vorzugsweise ein weicheres Material, beispielsweise TPE, auf oder besteht vorzugsweise vollständig aus diesem Material. Es ist also möglich, die Innenwand 11 ebenfalls aus einem harten Material auszugestalten und auf der Innenfläche 11 weichere Materialien einzulagern oder eine Schicht aus diesem weichen Material aufzubringen.

Vorzugsweise bestehen die Stege und Bereiche der Innenwand 11 oder deren gesamte Innenfläche 13 aus Kunststoff. Insbesondere wird bevorzugt, den Aufsatz 1 in einem Zwei-Komponenten-Spritzgussverfahren aus zwei Kunststoffmaterialien herzustellen, von denen eines hart und gegen Druck widerstandsfähig ist, während das andere weich ist und sich so an die Außenfläche des Fortsatzes anschmiegt, dass hohe Reibungskräfte aufgebaut werden.

Bei dem Ausführungsbeispiel gemäß Figur 1 sind alle Widerlager 25 innerhalb der Freiräume 11 gegenüber einer gedachten Mittelebene in Richtung auf einen der Stege versetzt. Der Aufsatz 1 gemäß Figur 1 ist daher so ausgebildet, dass hohe Widerstandskräfte gegen eine Verdrehung des Mantels 5 gegenüber der Innenwand 11 gegen den Uhrzeigersinn verhindert wird.

Denkbar ist es, bei dem Ausführungsbeispiel gemäß Figur 1 mindestens ein, vorzugsweise jedes zweite Widerlager 25 spiegelbildlich zu einer gedachten Mittelebene des Freiraums 31 anzuordnen. Auf diese Weise können hohe Widerstandskräfte aufgebaut werden, und zwar nicht nur bei einer Verdrehung, wie oben erläutert gegen den Uhrzeigersinn, sondern auch bei einer Verdrehung im Uhrzeigersinn.

Bei dem Ausführungsbeispiel gemäß Figur 2 sind einzelne Ringsegmente vorzusehen, die abwechselnd spiegelbildlich aufgebaut sind. Die Ringsegmente 33/1 weisen jeweils ein Widerlager 25 auf, das in einem Freiraum 31 angeordnet ist, und zwar versetzt zu einer gedachten Mittellinie nach rechts. Dadurch wirken die Widerlager der Ringsegmente 33/1 jeweils mit dem Steg 15/1a zusammen, sodass hohe Widerstandskräfte gegen ein Verdrehen des Aufsatzes 1 gegen den Uhrzeigersinn aufgebaut werden, wie dies anhand von Figur 1 erläutert wurde.

Da in den Ringsegmenten 31/2 ein spiegelbildlicher Aufbau vorliegt, wirken diese Ringsegmente 33/2 einer Verdrehung des Aufsatzes 1 nach dem Aufsetzen auf den Fortsatz einer Spritze, Karpule oder dergleichen im Uhrzeigersinn entgegen.

Durch die spiegelbildlich ausgebildeten Ringsegmente 33/1 und 33/2 wird folglich erreicht, dass dieser Aufsatz 1 gegen eine Verdrehung in beide Richtungen gesichert ist.

Betrachtet man das in Figur 4 wiedergegebene Ausführungsbeispiel des Aufsatzes 1, so zeigt sich, dass sechs Stege vorgesehen sind, von denen die Stege 15/1a und 15/1b einen Freiraum 31/1 umgrenzen, wobei dieser Freiraum in Richtung zur Mittelachse 17 durch die Innenwand 11 und radial nach außen durch die Innenfläche 24 des Mantels 5 abgeschlossen ist.

Versetzt zur Mittelebene des Freiraums 31/1 sind in der Nähe der Stege 15/1a und 15/1b spiegelbildlich zur gedachten Mittelebene des Freiraums 31/1 ausgerichtete Widerlager 25/1a und 25/1b vorgesehen, von denen das Widerlager 25/1a einen Basiskörper 27/1a aufweist, von dem ein in Richtung auf den Steg 15/1a weisender Vorsprung 29/1a ausgeht. Entsprechend weist der Vorsprung 29/1b zum Steg 15/1b. Bei einer Drehung gegen den Uhrzeigersinn wird der Steg 15/1a, wie anhand von Figur 1 erläutert, gestaucht, bis er letztlich radial ausgerichtet ist und eine maximale Druckkraft auf die Innenwand 11 ausübt. Eine weitere Drehung des Aufsatzes 1 wird dadurch verhindert, dass der Vorsprung 29/1a an dem Steg 15/1a anschlägt und eine weitere Verdrehung verhindert.

Bei einer Drehbewegung in die entgegengesetzte Richtung verhindert entsprechend der Vorsprung 29/1b eine Verschwenkung des Stegs 15/1b über die radiale Stellung hinaus, weil er an dem Steg anschlägt und eine weitere Verdrehung des Aufsatzes 1 gegenüber einem Fortsatz, auf den der Aufsatz 1 aufgesteckt ist, verhindert.

Das in Figur 4 dargestellte Ausführungsbeispiel ist also, wie jene gemäß Figur 2 gegen eine Verdrehung im Uhrzeigersinn als auch gegen eine Verdrehung gegen den Uhrzeigersinn gesichert.

## Patentansprüche

1. Aufsatz für eine Spritze oder Karpule mit
- einem Grundkörper (3), der einen Innenraum (7) umschließt, in den ein Fortsatz der Spritze oder Karpule einführbar ist, wobei
- der Grundkörper (3) einen den Innenraum (7) umgebenden Mantel (5) umfasst und mit
- einem mit dem Mantel (5) verbundenen, koaxial zu diesem angeordneten Innenkörper (9), der eine Innenwand (11) aufweist, die mit einer dem Innenraum (7) zugewandten Innenfläche (13) in aufgesetztem Zustand des Aufsatzes (1) an einer Außenfläche des Fortsatzes oder der Spritze oder Karpule anliegt, wobei
- die Innenwand (11) des Innenkörpers (9) über eine Anzahl von Stegen (15) mit dem Mantel (5) des Aufsatzes (1) verbunden ist,
**dadurch gekennzeichnet, dass**
- zumindest ein Steg (15) mit einer gedachten radial verlaufenden Linie (21) einen Winkel α einschließt, der größer 0° und spitz ist, so dass
- der zumindest eine Steg (15) bei Einleiten eines Drehmoments in den Aufsatz (1) so verschwenkbar ist, dass er radial verläuft und damit zwischen dem Mantel (5) und der Innenwand (11) des Innenkörpers (9) gestaucht wird.

2. Aufsatz nach Anspruch 1, **dadurch gekennzeichnet, dass** zwischen zwei benachbarten Stegen (15), dem Mantel (5) und der Innenwand (11) des Innenkörpers (9) ein Freiraum (31) eingeschlossen ist.

3. Aufsatz nach Anspruch 1 oder 2, **dadurch gekennzeichnet, dass** mindestens ein Widerlager (25) vorgesehen ist, das von der Innenseite (24) des Mantels (5) entspringt und in einem Abstand von einem Steg (15) angeordnet ist.

4. Aufsatz nach Anspruch 3, **dadurch gekennzeichnet, dass** das Widerlager (25) einen von der Innenseite (24) des Mantels (5) ausgehenden Basiskörper (27) aufweist, der in einem Abstand zur Innenseite (24) des Mantels (5) ein freies Ende aufweist.

5. Aufsatz nach Anspruch 4, **dadurch gekennzeichnet, dass** sich der Basiskörper (27) ausgehend von der Innenseite (24) des Mantels (5) in Richtung auf sein freies Ende verjüngt.

6. Aufsatz nach Anspruch 4 oder 5, **dadurch gekennzeichnet, dass** - vorzugsweise im Bereich des freien Endes - der Basiskörper (27) einen seitlichen Vorsprung (29) aufweist.

7. Aufsatz nach Anspruch 1, **dadurch gekennzeichnet, dass** zwei Widerlager (25) vorgesehen sind.

8. Aufsatz nach Anspruch 7, **dadurch gekennzeichnet, dass** die Widerlager (25) auf den einander abgewandten Außenseiten je mindestens einen Vorsprung (29) aufweisen.

9. Aufsatz nach Anspruch 2, **dadurch gekennzeichnet, dass** in einem Freiraum (31) ein oder zwei Widerlager (25) vorgesehen sind.

10. Aufsatz nach Anspruch 9, **dadurch gekennzeichnet, dass** zwischen zwei - in Umfangsrichtung aufeinander folgenden - Freiräumen (31) mit mindestens einem Widerlager (25) eine Lücke ohne Widerlager (25) vorgesehen ist.

11. Aufsatz nach Anspruch 2, **dadurch gekennzeichnet, dass** mindestens zwei - in Umfangsrichtung - in einem Abstand zueinander angeordnete Freiräume (31) vorgesehen sind.

12. Aufsatz nach Anspruch 11, **dadurch gekennzeichnet, dass** Ringsegmente gebildet werden, die jeweils mindestens einen Freiraum (31) umfassen, der von zwei benachbarten Stegen (15), dem Mantel (5) und der Innenwand (11) des Innenkörpers (9) eingeschlossen ist.

13. Aufsatz nach Anspruch 12, **dadurch gekennzeichnet, dass** ein Ringsegment zwei benachbarte Freiräume (31) aufweist.

14. Aufsatz nach einem der vorhergehenden Ansprüche 11 bis 13, **dadurch gekennzeichnet, dass** in mindestens einer Lücke zwischen zwei Freiräumen (31) wenigstens ein Widerlager (25) vorgesehen ist.

15. Aufsatz nach Anspruch 1, **dadurch gekennzeichnet, dass** die in Richtung der Mittelachse (17) des Grundkörpers (5) gemessene Höhe des Innenkörpers (9) kleiner ist als die des Grundkörpers (5).

## Claims

1. An attachment for a syringe or carpule, comprising
- a main body (3) that encloses an interior space (7), into which a protrusion of the syringe or carpule can be inserted, wherein
- the main body (3) comprises a jacket (5) surrounding the interior space (7), and
- comprising an inner body (9) connected to the jacket (5) and arranged coaxially thereto, the inner body having an inner wall (11) that, in the attached state of the attachment (1), contacts an outer surface of the protrusion or of the syringe or carpule by means of an interior surface (13) facing the interior space (7), wherein
- the inner wall (11) of the inner body (9) is connected to the jacket (5) of the attachment (1) by means of a number of webs (15),
**characterised in that**
- at least one web (15) encloses an angle α together with an imaginary radially extending line, the angle α being greater than 0° and acute, such that
- the at least one web (15) can be pivoted when a rotational torque is introduced in the attachment (1) such that it extends radially and is thus compressed between the jacket (5) and the inner wall (11) of the inner body (9).

2. The attachment according to claim 1, **characterised in that** an open space (31) is enclosed between two adjacent webs (15), the jacket (5) and the inner wall (11) of the inner body (9).

3. The attachment according to claims 1 or 2, **characterised in that** at least one abutment (25) is provided, which originates from the inner side (24) of the jacket (5) and is arranged at a distance from a web (5).

4. The attachment according to claim 3, **characterised in that** the abutment (25) comprises a base body (27) extending out from the inner side (24) of the jacket (5), which comprises a free end at a distance from the inner side (24) of the jacket (5).

5. The attachment according to claim 4, **characterised in that** the base body (27) tapers from the inner side (24) of the jacket (5) in the direction of its free end.

6. The attachment according to claim 4 or 5, **characterised in that** - preferably in the region of the free end - the base body (27) comprises a lateral projection (29).

7. The attachment according to claim 1, **characterised in that** two abutments (25) are provided.

8. The attachment according to claim 7, **characterised in that** the abutments (25) comprise at least one projection (29) on each of the outer sides facing away from one another.

9. The attachment according to claim 2, **characterised in that** one or two abutments (25) are provided in an open space (31).

10. The attachment according to claim 9, **characterized in that** between two circumferentially consecutive open spaces (31) comprising at least one abutment (25), a gap without an abutment (25) is provided.

11. The attachment according to claim 2, **characterised in that** at least two open spaces (31) are provided which are arranged at a distance from one another in a circumferential direction.

12. The attachment according to claim 11, **characterised in that** ring segments are formed which each comprise at least one open space (31), which is enclosed by two adjacent webs (15), the jacket (5) and the inner wall (11) of the inner body (9).

13. The attachment according to claim 12, **characterised in that** a ring segment comprises two adjacent open spaces (31).

14. The attachment according to any one of the preceding claims 11 to 13, **characterised in that** at least one abutment (25) is provided in at least one gap between two open spaces (31).

15. The attachment according to claim 1, **characterised in that** the height of the inner body (9) measured in the direction of the central axis (17) of the main body (5) is smaller than that of the main body (5).

## Revendications

1. Embout pour une seringue ou une carpule avec
- un corps de base (3) enfermant un espace intérieur (7) dans lequel un processus de la seringue ou de la carpule peut être inséré,
- le corps de base (3) comprenant une enveloppe (5) entourant l'espace intérieur (7), et avec
- un corps intérieur (9) relié avec l'enveloppe (5) et disposé de manière coaxiale avec celle-ci, le corps comprenant une paroi intérieure (11) qui repose, dans un état monté de l'embout (1), avec une surface intérieure (13) faisant face à l'espace intérieur (7) contre une surface extérieure du processus ou de la seringue ou de la carpule,
- la paroi intérieure (11) du corps intérieur (9) étant reliée avec l'enveloppe (5) de l'embout (1) par un nombre des âmes (15),
**caractérisé en ce que**
- au moins une âme (15) inclut un angle α supérieur à 0° et pointu avec une ligne radiale imaginaire (21), de telle manière que
- l'au moins une âme (15) peut être pivotée, en appliquant un couple sur l'embout (1), de telle manière qu'elle s'étend radialement et est ainsi comprimée entre l'enveloppe (5) et la paroi intérieure (11) du corps intérieur (9).

2. Embout selon la revendication 1, **caractérisé en ce qu'**un espace libre (31) est défini entre deux âmes adjacentes (15), l'enveloppe (5) et la paroi intérieure (11) du corps intérieur (9).

3. Embout selon la revendication 1 ou 2, **caractérisé en ce que** au moins une butée (25) est prévue, qui s'étend à partir de la face intérieure (24) de l'enveloppe (5) et est espacée d'une âme (15).

4. Embout selon la revendication 3, **caractérisé en ce que** la butée (25) comprend un corps de base (27) qui s'étend à partir de la face intérieure (24) de l'enveloppe (5) et présente une extrémité libre à distance de la face intérieure (24) de l'enveloppe (5).

5. Embout selon la revendication 4, **caractérisé en ce que** le corps de base (27) se rétrécit à partir de la face intérieure (24) de l'enveloppe (5) dans la direction vers son extrémité libre.

6. Embout selon la revendication 4 ou 5, **caractérisé en ce que** le corps de base (27) présente une saillie latérale (29), de préférence dans la région de l'extrémité libre.

7. Embout selon la revendication 1, **caractérisé en ce que** deux butées (25) sont prévues.

8. Embout selon la revendication 7, **caractérisé en ce que** les butées (25) respectivement présentent au moins une saillie (29) sur les faces extérieures détournées l'une de l'autre.

9. Embout selon la revendication 2, **caractérisé en ce qu'**une ou deux butées sont prévues dans un espace libre (31).

10. Embout selon la revendication 9, **caractérisé en ce qu'**un écart sans butée (25) est prévu entre deux espaces libres (31) consécutifs dans la direction circonférentielle avec au moins butée (25).

11. Embout selon la revendication 2, **caractérisé en ce que** au moins deux espaces libres (31) sont prévus qui sont espacés l'un de l'autre dans la direction circonférentielle.

12. Embout selon la revendication 11, **caractérisé en ce que** des segments d'anneau sont formés, chacun comprenant au moins un espace libre (31) enclavé par deux âmes adjacentes (15), l'enveloppe (5) et la paroi intérieure (11) du corps intérieur (9).

13. Embout selon la revendication 12, **caractérisé en ce qu'**un segment d'anneau comporte deux espaces libres (31) adjacents.

14. Embout selon l'une quelconque des revendications précédentes 11 à 13, **caractérisé en ce que** au moins une butée (25) est prévue dans au moins un écart entre deux espaces libres (31).

15. Embout selon la revendication 1, **caractérisé en ce que** la hauteur du corps intérieur (9) mesurée dans la direction de l'axe centrale (17) du corps de base (5) est inférieure à celle du corps de base (5).
